**Europäisches Patentamt**

**European Patent Office**

**Office européen des brevets**

(19)

(11) Numéro de publication: **0 193 451**
**B1**

(12) **FASCICULE DE BREVET EUROPEEN**

(45) Date de publication du fascicule du brevet: **19.09.90**

(21) Numéro de dépôt: **86400306.6**

(22) Date de dépôt: **12.02.86**

(51) Int. Cl.⁵: **C 07 D 303/04,**
C 07 D 301/06, C 07 D 301/32

(54) **Procédé perfectionné de fabrication d'oxydes terpéniques.**

(30) Priorité: **12.02.85 FR 8501960**

(43) Date de publication de la demande:
**03.09.86 Bulletin 86/36**

(45) Mention de la délivrance du brevet:
**19.09.90 Bulletin 90/38**

(84) Etats contractants désignés:
**AT BE CH DE GB IT LI LU NL SE**

(56) Documents cités:
**DE-A-2 803 791**
**FR-A-1 505 921**
**FR-A-1 572 146**

(73) Titulaire: **SOCIETE DE CONCEPTION ET
D'APPLICATIONS THERAPEUTIQUES (S.C.A.T.)**
**468 Chemin du Littoral**
**F-13321 Marseille Cedex (FR)**

(72) Inventeur: **Leseche, Bernard**
**2 Allée Guynemer**
**F-92330 Neuilly sur Marne (FR)**

(74) Mandataire: **Gutmann, Ernest et al**
**S.C. Ernest Gutmann - Yves Plasseraud 67,
boulevard Haussmann**
**F-75008 Paris (FR)**

Courier Press, Leamington Spa, England.

**Description**

L'invention est relative à des perfectionnements apportés au procédé de fabrication d'oxydes terpéniques à partir d'essences de térébenthines extraites de conifères et térébenthacées. Une matière préférée consiste en des essences de térébenthines de pins des Landes (Pinus pinaster) ou du Portugal (dont l'origine botanique est la même). Les produits d'oxydation de ces essences de térébenthines, plus particulièrement les oxydes terpéniques qui les composent, sont utilisés pour la constitution de préparations médicamenteuses utilisées pour le traitement, par exemple des voies respiratoires ou aériennes supérieures, ou pour la prévention des complications pulmonaires post-opératoires.

Il est connu que les procédés de fabrication de ces oxydes terpéniques comprennent des phases d'oxydation contrôlées, par l'air, de ces essences de térébenthine, en présence d'un catalyseur et, le cas échéant, de un ou plusieurs promoteurs assurant la transformation de la majeure partie de ces essences en oxydes terpéniques, puis des phases de rectification ou distillation fractionnée de ces oxydes terpéniques. Bien des améliorations ont déjà été apportées à ces procédés pour détruire les hydropéroxydes indésirables formés pendant les phases d'oxydation, voire pour en prévenir la formation. Les procédés mis en oeuvre jusqu'à ce jour ne permettent cependant pas le contrôle rigoureux de la fabrication dans toutes ses phases, en particulier n'empêchent pas les phénomènes de décomposition thermique du type craquage ou "cracking" qui s'observent à divers stades de la fabrication, notamment à divers stades des opérations de fractionnement visant à récupérer les oxydes terpéniques. En d'autres termes les conditions de distillation sont encore à ce jour précaires et aléatoires, et ce en raison de craquages qui s'observent au cours des fractionnements, d'une part, en fin de distillation, d'autre part, donc à deux périodes importantes de la distillation.

L'invention a donc pour but de remédier à ces difficultés, plus particulièrement de fournir un procédé permettant le contrôle rigoureux des différentes opérations de la production des oxydes terpéniques du genre en question, plus particulièrement de la prévention desdits phénomènes de craquage. Par conséquent l'invention a également pour but un accroissement des rendements de production en oxydes terpéniques utiles, avec pour corollaire la réduction des teneurs des compositions de carbures terpéniques obtenus en produits secondaires, du type α- et β-pinènes, limonène et paracymène, qui peuvent être causes d'effet secondaires, notamment d'allergies, au moins chez certains des patients absorbant les compositions médicamenteuses à base des susdits oxydes terpéniques.

Le procédé de fabrication selon l'invention qui comprend les phases d'oxydation contrôlée, puis les phases de rectification ou distillation fractionnée des oxydes terpéniques qui ont été rappelées plus haut, est caractérisé par la combinaison de caractéristiques que constituent, d'une part, la mise en contact du milieu de réaction avec des composés de bore insolubles ou insolubilisés pendant lesdites phases d'oxydation et, de préférence aussi, ladite phase de rectification ou distillation fractionnée et, d'autre part, la réalisation d'une opération de "mûrissement" du mélange oxydé, postérieurement aux phases d'oxydation et antérieurement aux phases de rectification, cette opération de mûrissement comprenant l'agitation du milieu de réaction porté à une température comprise entre environ 80°C et environ 100°C, sous pression atmosphérique, en présence d'air, pendant le temps nécessaire à transformer la majeure partie des hydropéroxydes présents ou formés, en cétones, alcools, aldéhydes ou autres produits oxygénés.

La combinaison de l'utilisation de catalyseurs constitués par des dérivés insolubilisés du bore et de la susdite opération de mûrissement a pour effet de supprimer les effets de craquage susmentionnés.

En ce qui concerne les conditions mêmes de réalisation des phases d'oxydation, on peut se reporter à la description du brevet français 1 572 146. Les composés du bore mis en oeuvre s'ajoutent aux catalyseurs métalliques déjà utilisés et constitués de préférence de sels solubles d'éléments de transition, tels que le nickel, le fer, le cuivre, le chrome, le manganèse ou de préférence le cobalt. On rappellera pour mémoire que des catalyseurs solubles préférés sont constitués par des abiétates de cobalt ou par les autres sels de cobalt qui ont également été identifiés dans le brevet français antérieur.

Les composés de bore insolubles ou insolubilisés mis en oeuvre, de préférence dès le début de la réaction d'oxydation, peuvent être constitués par tout composé insoluble ou insolubilisé du bore, tels que les oxydes de bore envisagés dans le brevet français 77 02748 ou le brevet allemand 28 03791. Des résultats particulièrement avantageux sont obtenus avec des borosilicates insolubles, par exemple du type de ceux connus sous la marque PYREX. De préférence le milieu de réaction est maintenu au contact de ces composés de bore insolubles ou insolubilisés pendant toute la durée des opérations s'étendant du début de l'oxydation jusqu'à la fin de la distillation. Le contact entre les milieux de réaction et le catalyseur peut être réalisé de toute façon appropriée. Par exemple les composés de PYREX se présentent sous forme de pièces ou éléments de barreaux introduits et maintenus dans le fond du réacteur. Le PYREX est d'un intérêt tout particulier, en ce qu'il peut se présenter sous toute forme désirée, par exemple de petits cylindres pleins. Outre les composés du bore, il contient des silicates permettant une vitrification de ces composés du bore. Il va de soi que tout autre produit susceptible d'être associé avec du bore dans des conditions analogues peut être utilisé. Il avantageux d'utiliser le PYREX sous forme de petits barreaux cylindriques ou de billes susceptibles de former un tapis de PYREX® au fond du réacteur.

Pour obtenir les résultats favorables recherchés, l'utilisation des susdits catalyseurs insolubles ou insolubilisés de bore est combinée avec la phase de mûrissement dans les conditions qui ont déjà été indiquées. Avantageusement la durée de ce mûrissement est d'au moins 30 minutes. Les températures limites indiquées peuvent naturellement varier selon les charges d'essences de térébenthines utilisées ou encore selon les natures des essences de térébenthines utilisées. D'une façon générale la température de mûrissement sera suffisante, environ 80°C, pour que soit assurée la destruction des péroxydes au fur et à mesure de leur formation, la température ne devant cependant pas excéder celle, environ 180°C, qui aurait pour effet la production d'eau oxygénée en excès, laquelle pourrait à son tour être génératrice de réactions parasites.

L'utilisation du procédé selon l'invention assure également des conditions de distillation qui peuvent être parfaitement contrôlées. De façon en soi connue, ces opérations de distillation sont réalisées sous pression réduite, les intervalles de températures pendant lesquels passent les oxydes terpéniques étant évidemment dépendants de la pression réduite maintenue dans l'installation.

De préférence le fractionnement est réalisé sous une pression réduite de 1066,58 Pa (8 mm de mercure), les produits utiles distillant alors entre environ 70°C (température dans le haut de la colonne de distillation) et 110-115°C. Ces conditions opératoires ne sont pas les seules susceptibles d'être utilisées. Par exemple, les produits utiles passent entre une température minimum en haut de colonne de 65-75°C et une température finale de 114-123°C, lorsque la distillation est effectuée sous une pression partielle de 1599,86 Pa (12 mm de mercure), ou encore entre une température minimum d'environ 85-95°C et une température maximum d'environ 128-137°C, lorsque la distillation est réalisée sous une pression réduite de 3333,05 Pa (25 mm de mercure), ou encore entre une température minimum de 45-50°C et une température maximum de 86-91°C, lorsque l'on opère la distillation sous une pression réduite de 266,64 Pa (2 mm de mercure). Quelles que soient les conditions de température et de pression réduite utilisées pour la distillation, il est important de noter que désormais l'élimination des craquages permet le réglage précis des conditions de distillation et en particulier de toujours terminer la distillation sous un vide constant. Il en résulte par conséquent une amélioration de la reproductibilité des proportions relatives différentes d'oxydes terpéniques contenus dans les compositons obtenues en fin de traitement d'une même matière première.

En particulier, partant des essences de térébenthines des Landes ou du Portugal mentionnées plus haut, le procédé selon l'invention conduit à des compositions d'oxydes terpéniques dont les teneurs finales en alpha- et bêta-pinènes, limonène et paracymène sont au plus égales à 5-6%, dont l'indice d'acide ne dépasse pas 5 et dont enfin les taux en hydropéroxydes sont quasiment nuls.

Comme il va de soi et comme il résulte d'ailleurs déjà de ce qui précède, l'invention ne se limite nullement à ceux de ses modes d'application et de réalisation qui ont été spécialement envisagés; elle en embrasse, au contraire, toutes les variantes.

## Revendications

1. Procédé de fabrication d'oxydes terpéniques comprenant des phases d'oxydation contrôlée d'essences de thérébentines par l'air en présence d'un catalyseur et, le cas échéant, de promoteurs assurant la transformation de la majeure partie de ces essences de thérébentines en oxydes terpéniques, et ultérieurement des phases de rectification ou de distillation fractionnée des oxydes terpéniques formés, ledit procédé est caractérisé par la combinaison des caractéristiques que constituent, d'une part, la mise en contact du milieu de réaction avec des composés de bore insolubles ou insolubilisés pendant lesdites phases d'oxydation et, de préférence aussi pendant ladite phase de rectification ou distillation fractionnée et, d'autre part, la réalisation d'une opération de "mûrissement" du mélange oxydé, postérieurement aux phases d'oxydation et antérieurement aux phases de rectification, cette opération de mûrissement comprenant l'agitation du milieu de réaction porté à une température comprise entre environ 80°C et environ 100°C en présence d'air pendant le temps nécessaire à transformer la majeure partie des hydroperoxydes présents ou formés, en cétones, alcools, aldéhydes ou autres produits oxygénés.

2. Procédé selon la revendication 1, caractérisé en ce que les composés insolubles ou insolubilisés du bore sont des oxydes de bores vitrifiés.

3. Procédé selon la revendication 1, caractérisé en ce que les composés du bore sont des borosilicates, du type PYREX® ou analogue.

4. Procédé selon l'une quelconque des revendications 1 à 4, caractérisé en ce que le mûrissement est maintenu pendant une durée d'au moins 30 minutes.

5. Procédé selon l'une quelconque des revendications 1 à 4, caractérisé en ce que la phase de distillation fractionnée est réalisée sous pression réduite.

6. Procédé selon la revendication 5, caractérisé en ce que la distillation est réalisée sous une pression réduite d'environ 1066,58 Pa (8mm de Hg) et en ce que l'on recueille les oxydes de terpènes qui distillent entre environ 70°C et environ 110-115°C.

7. Procédé selon la revendication 5, caractérisé en ce que la distillation est réalisée sous une pression réduite d'environ 1599,86 Pa (12 mm de Hg) et en ce que l'on recueille les oxydes de terpènes qui distillent entre environ 65-75°C et 114-123°C.

8. Procédé selon la revendication 5, caractérisé en ce que la distillation est réalisée sous une pression réduite d'environ 3333,05 Pa (25 mm de Hg) et en ce que l'on recueille les oxydes de terpènes qui distillent entre environ 85-95°C et 128-137°C.

9. Procédé selon la revendication 5, caractérisé en ce que la distillation est réalisée sous une pression réduite d'environ 266,64 Pa (2 mm de Hg) et en ce que l'on recueille les oxydes de terpènes qui distillent entre 45-50°C et 80-91°C.

10. Procédé selon l'une quelconque des revendications 1 à 9, caractérisé en ce que les essences de térébenthines initialement mises en oeuvre sont des essences de pins, notamment des Landes ou du Portugal.

**Patentansprüche**

1. Verfahren zur Herstellung von Terpenoxiden mit den Stufen der kontrollierten Oxydation von Terpentinölen durch Luft in Gegenwart eines Katalysators und gegebenenfalls von Promotoren, die die Umwandlung des Hauptteils dieser Terpentinöle in Terpenoxide gewährleisten, und dann Stufen der Rektification oder der fraktionierten Destillation der gebildeten Terpenoxide, gekennzeichnet durch die Kombination der Merkmale, die einerseits aus der Kontaktierung des Reaktionsmediums während der Oxydationsstufen und vorzugsweise auch während der Stufe der Rektification oder fraktionierten Destillation mit Borverbindungen, die unlöslich oder unlöslich gemacht worden sind, und andrerseits aus der Durchführung eines "Reifungs"-Vorgangs des oxydierten Gemischs nach den Oxydationstufen und vor den Rektifikationsstufen bestehen, wobei in diesem Reifungsvorgang das auf etwa 80°C bis etwa 100°C gebrachte Reaktionsmedium in Gegenwart von Luft während einer genügend langen Zeit bewegt wird, um den Hauptteil der vorhandenen oder gebildeten Hydroperoxide in Ketone, Alkohole, Aldehyde oder andere oxydierte Produkte umzuwandeln.

2. Verfahren nach Anspruch 1, dadurch gekennzeichnet, daß die unlöslichen oder unlöslich gemachten Borverbindungen zu Glas verarbeitbare Boroxide sind.

3. Verfahren nach Anspruch 1, dadurch gekennzeichnet, daß die Borverbindungen Borsilicate des Typs Pyrex® oder ähnlich sind.

4. Verfahren nach einem der Ansprüche 1 bis 3, dadurch gekennzeichnet, daß die Reifung während einer Zeit von mindestens 30 min durchgeführt wird.

5. Verfahren nach einem der Ansprüche 1 bis 4, dadurch gekennzeichnet, daß die Stufe der fraktionierten Destillation unter vermindertem Druck durchgeführt wird.

6. Verfahren nach Anspruch 5, dadurch gekennzeichnet, daß die Destillation unter einem vermindertem Druck von etwa 1066,58 Pa (8 mm Hg) durchgeführt wird und die Terpenoxide, die bei etwa 70°C bis etwa 110—115°C destillieren, gesammelt werden.

7. Verfahren nach Anspruch 5, dadurch gekennzeichnet, daß die Destillation bei einem vermindertem Druck von etwa 1599,86 Pa (12 mm Hg) durchgeführt wird und die Terpenoxide, die bei etwa 65—75°C bis 114—123°C destillieren, gesammelt werden.

8. Verfahren nach Anspruch 5, dadurch gekennzeichnet, daß die Destillation bei einem vermindertem Druck von etwa 3333,05 Pa (25 mm Hg) durchgeführt wird und die Terpenoxide, die bei etwa 85-95°C bis 128—137°C destillieren, gesammelt werden.

9. Verfahren nach Anspruch 5, dadurch gekennzeichnet, daß die Destillation bei einem vermindertem Druck von etwa 266,64 Pa (2 mm Hg) durchgeführt wird unf die Terpenoxide, die bei 45—50°C bis 80—91°C destillieren, gesammelt werden.

10. Verfahren nach einem der Ansprüche 1 bis 9, dadurch gekennzeichnet, daß die am Anfang eingesetzten Terpentinöle Kiefernöle, insbesondere von Kiefern der Landes oder aus Portugal, sind.

**Claims**

1. Process for the production of terpenic oxides comprising controlled oxidation phases of essences of terpentine by air in the presence of a catalyst and, if necessary, promotors ensuring the transformation of a major portion of the essences of terpentine into terpenic oxides, and subsequently rectification or fractional distillation of the formed terpenic oxides, the said process being characterised by the combination of the characteristics constituted by, on the one hand the contacting of the reaction medium with insoluble or insolubilised borium compounds during the said oxidation phases and preferably also during the said rectification or fractional distillation phase and, on the other hand the carrying out of an "ageing" operation on the oxidised mixture subsequent to the oxidation phases and preceding the rectification phases, this ageing operation comprising the agitation of the reaction mixture brought to a temperature between about 80°C and about 100°C in the presence of air for a time sufficient to transform a major portion of the hydroperoxides present or formed into ketones, alcohols, aldehydes or other oxygenated products.

2. Process according to claim 1 characterised in that the insoluble or insolubilised borium compounds are vitrified borium oxides.

3. Process according to claim 1 characterised in that the compounds of borium are borosilicates, of the PYREX® type or analogues.

4. Process according to any one of claims 1 to 3 characterised in that the ageing is maintained for a period of at least 30 minutes.

5. Process according to any one of claims 1 to 4 characterised in that the fractional distillation phase is carried out under reduced pressure.

6. Process according to claim 5 characterised in that the distillation is carried out under a reduced

pressure of about 1066,58 Pa (8mm of Hg) and in that the terpenic oxides distilling between about 70°C and about 110-115°C are collected.

7. Process according to claim 5 characterised in that the distillation is carried out under a reduced pressure of about 1599,86 Pa (12mm of Hg) and in that the terpenic oxides distilling between about 65-75°C and 114-123°C are collected.

8. Process according to claim 5 characterised in that the distillation is carried out under a reduced pressure of about 3333,05 Pa (25mm of Hg) and in that the terpenic oxides distilling between about 85-95°C and 128-137°C are collected.

9. Process according to claim 5 characterised in that the distillation is carried out under a reduced pressure of about 266,64 pa (2mm of Hg) and in that the terpenic oxides distilling between 45-50°C and 80-91°C are collected.

10. Process according to anyone of claims 1 to 9 characterised in that the terpentine essences initially used are essences of pine, particularly pine of the Landes or pine of Portugal.